# EUROPEAN PATENT SPECIFICATION

(11) **EP 2 557 159 B1**
(45) Date of publication and mention of the grant of the patent: **24.08.2016**
(21) Application number: 11762858.6
(22) Date of filing: 29.03.2011
(51) Int. Cl.: C12N 15/09, A61K 31/7105, A61K 31/713, A61K 39/395, A61K 45/00, A61K 48/00, C12Q 1/02, C12Q 1/68, G01N 33/48, G01N 33/53, G01N 33/574

(54) **PROGNOSTIC METHOD FOR PULMONARY ADENOCARCINOMA, PULMONARY ADENOCARCINOMA DETECTION KIT, AND PHARMACEUTICAL COMPOSITION FOR TREATING PULMONARY ADENOCARCINOMA**
VERFAHREN ZUR PROGNOSE VON LUNGEN-ADENOKARZINOMEN, KIT ZUM NACHWEIS VON LUNGEN-ADENOKARZINOMEN UND PHARMAZEUTISCHE ZUSAMMENSETZUNG ZUR BEHANDLUNG VON LUNGEN-ADENOKARZINOMEN
MÉTHODE DE PRONOSTIC D'ADÉNOCARCINOME PULMONAIRE, KIT DE DÉTECTION D'ADÉNOCARCINOME PULMONAIRE ET COMPOSITION PHARMACEUTIQUE POUR LE TRAITEMENT D'UN ADÉNOCARCINOME PULMONAIRE

(30) Priority: 30.03.2010 JP 2010078772
(43) Date of publication of application: 13.02.2013
(73) Proprietor: Japan Health Sciences Foundation, Tokyo 1010032 (JP)
(72) Inventor: HONDA Kazufumi, Tokyo 104-0045 (JP); YAMADA Tesshi, Tokyo 104-0045 (JP); NORO Rintaro, Tokyo 104-0045 (JP); HIROHASHI Setsuo, Tokyo 104-0045 (JP)
(74) Representative: Epping - Hermann - Fischer
(86) International application number: PCT/JP2011/057866
(87) International publication number: WO 2011/122634

(56) References cited:
- JP-A- 2009 100 737
- OKUMURA M ET AL: "Candidates for tumor-specific alternative splicing", BIOCHEMICAL AND BIOPHYSICAL RESEARCH COMMUNICATIONS, ACADEMIC PRESS INC. ORLANDO, FL, US, vol. 334, no. 1, 19 August 2005 (2005-08-19), pages 23-29, XP027230083, ISSN: 0006-291X, DOI: 10.1016/J.BBRC.2005.06.057 [retrieved on 2005-07-12]
- YAMADA S ET AL: "RNAi-mediated down-regulation of +/--actinin-4 decreases invasion potential in oral squamous cell carcinoma", INTERNATIONAL JOURNAL OF ORAL AND MAXILLOFACIAL SURGERY, COPENHAGEN, DK, vol. 39, no. 1, 1 January 2010 (2010-01-01), pages 61-67, XP026856624, ISSN: 0901-5027 [retrieved on 2009-11-13]
- HONDA K ET AL: "Actinin-4, a novel actin-bundling protein associated with cell motility and cancer invasion", THE JOURNAL OF CELL BIOLOGY : JCB, THE ROCKEFELLER UNIVERSITY PRESS, US, vol. 140, no. 6, 23 March 1998 (1998-03-23), pages 1383-1393, XP002217225, ISSN: 0021-9525, DOI: 10.1083/JCB.140.6.1383
- YAMAGATA N. ET AL.: 'A training-testing approach to the molecular classification of resected non-small cell lung cancer.' CLIN. CANCER RES. vol. 9, no. 13, 2003, pages 4695 - 4704, XP055070065
- YASUO UESHIMA ET AL.: 'CEA Yosei Hishosaibo Haigan no Kento (LP2-21)' JPN J. THORAC. CARDIOVASC. SURG. vol. 50, September 2002, page 443
- YAMADA S. ET AL.: 'RNAi-mediated down-regulation of alpha-actinin-4 decreases invasion potential in oral squamous cell carcinoma.' INT. J. ORAL MAXILLOFAC. SURG. vol. 39, no. 1, January 2010, pages 61 - 67, XP026856624
- Sohei Yamamoto ET AL: "Actinin-4 gene amplification in ovarian cancer: a candidate oncogene associated with poor patient prognosis and tumor chemoresistance", Modern Pathology, vol. 22, no. 4, 16 January 2009 (2009-01-16), pages 499-507, XP055119089, ISSN: 0893-3952, DOI: 10.1038/modpathol.2008.234
- Noboru Yamagata ET AL: "2003;9:4695-4704. Clin Cancer Res of Resected Non-Small Cell Lung Cancer A Training-Testing Approach to the Molecular Classification Updated version", Clin Cancer Res, vol. 9, 15 October 2003 (2003-10-15), pages 4695-4704, XP055119106,

## Description

### Technical Field

The present invention relates to the use of a pulmonary adenocarcinoma detection kit.

### Background Art

Clinical stage I lung adenocarcinoma is an early lesion that has not yet metastasized to sites other than the primary focus. According to the current classification, stage I lung adenocarcinoma is further classified into stage IA (the diameter of tumor of less than 3 cm) and stage IB (the diameter of tumor of 3 cm or larger). According to Kato et al., the postoperative five-year survival rate of clinical stage I lung adenocarcinoma is 85%, and it has been verified that postoperative chemotherapy (oral administration of UFT for two years) significantly contributes to improving the prognosis in stage I lung adenocarcinoma (Non-Patent Document 1: Kato H, Ichinose Y, Ohta M, Hata E, Tsubota N, Tada H, Watanabe Y, Wada H, Tsuboi M, Hamajima N, Ohta M; Japan Lung Cancer Research Group on Postsurgical Adjuvant Chemotherapy. A randomized trial of adjuvant chemotherapy with uracil-tegafur for adenocarcinoma of the lung; N. Engl. J. Med., 2004 Apr 22; 350 (17): 1713-21). Currently, it is recommended that postoperative chemotherapy be given to patients with stage I lung adenocarcinoma; however, not all the patients with stage I lung adenocarcinoma are benefited from the postoperative chemotherapy. Accordingly, it is considered that if postoperative prognosis is predicted, a risk that unnecessary chemotherapy is given to a group of patients with favorable prognosis can be reduced.

A molecule, α-actinin-4, has been isolated as an actin-binding protein involved in the infiltration and metastasis of a cancer cell (Non-Patent Document 2: Honda et al., J Cell Biol. 1998 Mar 23; 140 (6): 1383-93). Further, it has been reported that an expression of α-actinin-4 protein is involved in the cell movement and infiltration property (Non-Patent Document 3: Honda et al., Gastroenterology. 2005 Jan; 128 (1): 51-62). Furthermore, candidates for tumor-specific alternative splicing were discussed (Non-Patent Document 4: Okumura M. et al., Biochemical and Biophysical Research Communications. 2005; 334 (1): 23-29). It was also found that RNAi-mediated down-regulation of α-actinin-4 decreases invasion potential in oral squamous cell carcinoma (Non-Patent Document 5: Yamada S. et al., International Journal of Oral and Maxillofacial Surgery. 2010; 39 (1): 61-67). Moreover, a training-testing approach to the molecular classification of resected non-small cell lung cancer has been reported. (Non-Patent Document 6: Yamagata N. et al., Clin. Cancer Res. 2003; 9 (13): 4695-4704). Furthermore, an actinin-4 gene amplification in ovarian cancer has been described (Non-Patent Document 7: Yamamoto S. et al., Modem Pathology 2009; 22 (4): 499-507). Finally, a diagnostic method of cancer, which uses copy number or expression level of α-actinin-4 gene as a marker was reported (JP 2009 100737 A).

### Prior Art Literature

### Non-Patent Literature

Non-Patent Document 1: Kato H. et al., N. Engl. J. Med., 2004 Apr 22; 350(17): 1713-21
Non-Patent Document 2: Honda et al., J Cell Biol. 1998 Mar 23; 140 (6): 1383-93
Non-Patent Document 3: Honda et al., Gastroenterology. 2005 Jan; 128 (1): 51-62
Non-Patent Document 4: Okumura M. et al., Biochemical and Biophysical Research Communications. 2005; 334 (1): 23-29
Non-Patent Document 5: Yamada S. et al., International Journal of Oral and Maxillofacial Surgery. 2010; 39 (1): 61-67
Non-Patent Document 6: Yamagata N. et al., Clin. Cancer Res. 2003; 9 (13): 4695-4704
Non-Patent Document 7: Yamamoto S. et al., Modem Pathology 2009; 22 (4): 499-507

### Patent-Literature

JP 2009 100737 A: Akio H. et al.

### Summary of Invention

### Problems to be Solved by the Invention

The present inventors have found that patients with an enhanced α-actinin-4 (ACTN4) protein expression in a stage I lung adenocarcinoma tissue exhibit a shorter overall survival (OS) than do patients without enhanced ACTN4 expression with statistically significant difference. The present inventors have also found that patients in a group having an increased copy number of α-actinin-4 protein (ACTN4) gene in the genome of a stage I lung adenocarcinoma tissue cell exhibit a shorter OS than do patients in a group not having an increased copy number of the gene. The present inventors also found that OS of patients with stage I lung adenocarcinoma can be more precisely predicted by a combination of a copy number of ACTN4 gene and an expression level of α-actinin-4 protein compared to an independent analysis conducted with respect to each of the copy number of ACTN4 gene and the expression level of α-actinin-4 protein. The present invention has been accomplished based on the findings as described above.

Accordingly, the present invention is drawn to the use of a pulmonary adenocarcinoma detection kit. In addition a prognostic method for pulmonary adocarcinoma and a pharmaceutical composition for treating pulmonary adenocarcinoma are not claimed, but described.

### Means for Solving the Problem

The present invention is drawn to the use of a kit, comprising either
- a probe that is available for determining the copy number of ACTN4 gene in a cell
- or a reagent for quantitative determination of α-actinin-4 protein in the tissue or cell
- for in vitro detecting a poor-prognosis stage I lung adenocarcinoma in a mammal, as defined in the claims set out below.
Not claimed, but described is a diagnostic method for detecting poor-prognosis stage I lung adenocarcinoma in a mammal, including a step of measuring the copy number of α-actinin-4 gene in a stage I lung adenocarcinoma tissue cell obtained from the aforementioned mammal. According to the present method, when the copy number of the aforementioned gene exceeds four, it is indicated that the stage I lung adenocarcinoma developed in the mammal is poor-prognosis stage I lung adenocarcinoma.

The present invention also refers to the use of a kit for in vitro detecting a poor-prognosis stage I lung adenocarcinoma in a mammal, comprising a reagent that is available for determining the copy number of ACTN4 gene in a cell.

Described is a diagnostic method for detecting poor-prognosis stage I lung adenocarcinoma in a mammal, including the steps of (a) measuring an expression level of α-actinin-4 protein in a stage I lung adenocarcinoma tissue or cell obtained from the aforementioned mammal and (b) comparing the expression level obtained in the step (a) with an expression level of α-actinin-4 gene in a corresponding normal tissue or cell. According to the present method, when an expression of the aforementioned protein or gene in the aforementioned stage I lung adenocarcinoma tissue or cell is enhanced, it is indicated that the stage I lung adenocarcinoma developed in the mammal is poor-prognosis stage I lung adenocarcinoma.

The present invention also refers to the use of a kit for in vitro detecting a poor-prognosis stage I lung adenocarcinoma in a mammal, comprising a reagent that can measure the expression level of ACTN4 gene in the tissue or cell.

Moreover, a pharmaceutical composition for treating stage I lung adenocarcinoma, containing an α-actinin-4 inhibitor and a pharmaceutically acceptable carrier is described.

The present invention also refers to the use of a kit for in vitro measuring the copy number of ACTN4 gene in human cells, comprising a probe specific for the gene on genome that can be used in a FISH method, wherein the specific probe has the nucleotide sequence of the region from 43.81 Mb to 44.02 Mb on human chromosome 19.

### Effect of the Invention

According to the present invention, prediction of prognosis of stage I lung adenocarcinoma becomes possible, and therefore appropriate therapy can be selected. For example, when stage I lung adenocarcinoma is determined as poor-prognosis stage I lung adenocarcinoma according to the present invention, chemotherapy using a medicine such as a combination preparation of tegafur and uracil, and cisplatin is recommended after extirpation of the adenocarcinoma. On the other hand, when stage I lung adenocarcinoma is not determined as poor-prognosis stage I lung adenocarcinoma according to the present invention, unnecessary chemotherapy can be avoided. Furthermore, according to the present invention, means for treating stage I lung adenocarcinoma, for example, poor-prognosis stage I lung adenocarcinoma, is provided.

### Brief Description of Drawings

FIGS. 1(A) and 1(B) are graphs showing survival curves for the two groups, as prepared by the Kaplan-Meier method, positive cases and negative cases of acceleration of α-actinin-4 protein expression in lung adenocarcinoma tissue (stages I and IA) (cohort 1).
FIGS. 2(A) and 2(B) are graphs showing survival curves for two groups, as prepared by Kaplan-Meier method, positive cases and negative cases of acceleration of α-actinin-4 protein expression in lung adenocarcinoma tissue (stages I and IA) (cohort 2).
FIGS. 3(A) and 3(B) are graphs showing survival curves for three groups, stage I lung adenocarcinoma patients, prepared by the Kaplan-Meier method: a group of a serum CEA of 5 ng/mL or less and actinin-4 negative; a group of a serum CEA of more than 5 ng/mL or α-actinin-4 protein positive; and a group of a serum CEA of more than 5 ng/mL and α-actinin-4 protein positive.
FIG. 4 is a graph showing survival curves for the three groups, the stage I lung adenocarcinoma patients as prepared by the Kaplan-Meier method: a group of ACTN4 amplification (-) and α-actinin-4 protein expression negative; a group of ACTN4 gene amplification (-) and α-actinin-4 protein expression positive; and a group of ACTN4 gene amplification (+) and α-actinin-4 protein expression positive.
FIG. 5 contains photo-micrographs showing expression in lung adenocarcinoma cells with immunohistochemical staining.
FIGS. 6(A) and 6(B) are graphs showing survival curves for the two groups, as prepared by the Kaplan-Meier method, positive cases and negative cases of acceleration of α-actinin-4 protein expression in lung adenocarcinoma tissue (stages I to III and stage I) (cohort 1 and 2).
FIG. 7 contains photo-micrographs of lung adenocarcinoma cells labeled by the fluorescent in situ hybridization (FISH) method.
FIGS. 8(A) and 8(B) are graphs showing survival curves for the two groups, as prepared by the Kaplan-Meier method, positive cases and negative cases of tissue microarrays in lung adenocarcinoma tissue (stage I).
FIGS. 9(A), 9(B), 9(C) and 9(D) are graphs showing survival curves for the four groups, as prepared by the Kaplan-Meier method, positive cases and negative cases of acceleration of α-actinin-4 protein expression and tissue microarrays in lung adenocarcinoma tissue (stages I and IA) (cohort 1 and 2).

### Mode for Carrying Out the Invention

Cancer to be treated by the present invention is stage I lung adenocarcinoma. Lung cancer is roughly classified into non-small cell carcinoma and small cell carcinoma. Lung adenocarcinoma is classified into non-small cell carcinoma. Stage I lung adenocarcinoma is the earliest stage of lung adenocarcinoma, which has not yet metastasized to a lymph node and other organs. According to the described diagnostic method, whether or not stage I lung adenocarcinoma developed in a mammal is poor-prognosis can be determined. Further, a disease to be treated by the present invention is stage I lung adenocarcinoma, preferably poor-prognosis stage I lung adenocarcinoma. In another preferred embodiment, a disease to be treated by the present invention is stage I lung adenocarcinoma exhibiting amplified ACTN4 genes or an enhanced expression of the gene in the carcinoma cells, particularly, stage I lung adenocarcinoma determined as poor-prognosis according to the diagnostic method described.

In the present specification, the term "poor-prognosis" means that, for example, a five-year survival rate after excision of cancer is less than 90%, preferably less than 85%, more preferably less than 70%, and most preferably less than 60%. Also, "poor-prognosis" in the present specification means that chemotherapy is required after excision of cancer. Therefore, according to another embodiment of the described diagnostic method, the method is for detecting stage I lung adenocarcinoma requiring postoperative chemotherapy in a mammal or a method for determining whether or not a patient with stage I lung adenocarcinoma requires postoperative chemotherapy.

The "α-actinin-4" or "ACTN4", which is used as an index of diagnosis and targeted in a describedtreatment, can be α-actinin-4 (ACTN4 gene product) derived from a mammal subjected to diagnosis or treatment. The ACTN4 gene sequence and the sequence of amino acid encoded by the gene are known in the art, and examples thereof include the sequences of human-derived ACTN4 shown in SEQ ID NOs: 1 and 2 (Ensembl ID:ENSG00000130402 and ENST00000252699). Sequences of human-derived ACTN4 are also registered as NCBI accession numbers: NM_004924 and NP_004915. Further, the gene locus of the ACTN4 gene is also known in the art, for example, the human ACTN4 gene is located on chromosome 19 between base positions 43,830,167 and 43,913,010. With respect also to mammalian animals other than humans, similar genes and proteins corresponding to human-derived ACTN4 are available.

In the present specification, it is verified that amplified ACTN4 genes (an increased copy number in the genome) and an enhanced expression of α-actinin-4 protein, which is a transcription product of the gene, are poor-prognosis factors for stage I lung adenocarcinoma. Further, it is verified that a combination of amplified ACTN4 genes and an enhanced expression of ACTN4 protein are poor-prognosis factors based on which prognosis of stage I lung adenocarcinoma can be more precisely predicted compared to an independent analysis conducted with respect to each one of them. Accordingly, by examining the copy number of ACTN4 gene in a stage I lung adenocarcinoma tissue cell obtained from a subject mammal (humans or mammals other than humans), whether or not the stage I lung adenocarcinoma is poor-prognosis can be diagnosed. Further, by examining an enhanced expression of α-actinin-4 protein in a stage I lung adenocarcinoma tissue or cell obtained from the aforementioned mammal, whether or not the stage I lung adenocarcinoma is poor-prognosis can also be diagnosed.

A stage I lung adenocarcinoma tissue or cell subjected to a diagnosis can be an operative sample extirpated from a patient by surgery or a biopsy sample obtained by endoscopy and the like.

In a described diagnostic method, whether or not stage I lung adenocarcinoma is poor-prognosis is determined by examining the copy number of ACTN4 gene in a stage I lung adenocarcinoma tissue cell, whereby poor-prognosis stage I lung adenocarcinoma is detected. The above method includes the step of measuring the copy number of ACTN4 gene in a stage I lung adenocarcinoma tissue cell. When the copy number exceeds 4, stage I lung adenocarcinoma in a subject mammal is determined (diagnosed) as poor-prognosis stage I lung adenocarcinoma.

A publicly known, standardized method in the art can be used to measure the copy number of ACTN4 gene. The ACTN4 gene sequence and the sequence of an amino acid encoded by the gene are as described above, and a person skilled in the art can measure the copy number of ACTN4 gene using a standardized method with reference to these sequences. Examples of a method for measuring the copy number of the gene include, particularly, an assay based on hybridization and amplification.

Examples of the assay based on hybridization include Southern blotting. In this method, genomic DNA is fragmented and separated by electrophoresis, and transferred to a membrane and hybridized with an ACTN4-specific probe. A relative copy number of ACTN4 gene is obtainable by comparing the hybridization signal intensity emitted by the specific probe with the signal intensity emitted by a control probe, which will hybridize with a region in which the copy number of the gene is not increased in the same genome.

According to a preferred embodiment of the present invention, measurement of the copy number of ACTN4 gene is performed by florescence in situ hybridization (FISH), which uses a probe specific to the gene in the genome (refer to Angerer, Meth. Enzymol. 152, 649, 1987). Generally, in situ hybridization is mainly performed as follows; a tissue or cell to be analyzed is immobilized and subjected to pre-hybridization treatment. Then, a probe specific to the nucleic acid of the tissue or cell is hybridized, followed by a wash to remove an unbound specific probe. Subsequently, a hybridized specific probe is detected. The specific probe used in the above-described application is labeled with a fluorescence reporter. The copy number of ACTN4 gene in a cell is obtainable by counting the number of fluorescence in the nucleus of the cell.

According to a particularly preferred embodiment of the present invention, the aforementioned specific probe used for FISH consists of a nucleotide sequence of a region from 43.81 Mb to 44.02 Mb on human chromosome 19. A probe consisting of the nucleotide sequence of the above region is particularly effective in measuring the copy number of ACTN4 gene in the genome, which enables accurate measurement with high sensitivity. Therefore, according to another embodiment of the present invention, the use of a kit for measuring the copy number of α-actinin-4 gene in a human cell is provided. The kit includes a probe specific to the aforementioned gene in the genome that can be used for FISH, and the specific probe consists of the nucleotide sequence of the region from 43.81 Mb to 44.02 Mb on human chromosome 19. The specific probe may be a chain of nucleotide molecules or a combination of two or more (preferably two) chains of nucleotide molecules covering the region from 43.81 Mb to 44.02 Mb on human chromosome 19.

As another assay based on hybridization, comparative genomic hybridization (CGH) can also be used. In CGH, DNA is extracted from each of a test cell to be analyzed and a normal cell to be used as a control, and DNA obtained from each cell is separately labeled with a fluorescent dye of different color. A mixed solution containing equivalent amount of each labeled DNA is prepared and kept at 37°C for two to three days in the dark to allow test DNA and control DNA to competitively bind to all the sites in chromosomes. As a result, if the copy number of the gene is increased in the test DNA, fluorescence of the color labeled with the test DNA is intensely observed at a position corresponding to the gene on a slide. In contrast, if the copy number of the gene is decreased in the test DNA, fluorescence of the color labeled with the control DNA is intensely observed at a position corresponding to the gene on a slide.

An assay based on amplification includes quantitative nucleic acid amplification. In this method, because the ACTN4 gene in the genome is a template in an amplification reaction, the amplification product is obtained in an amount in proportion to the copy number of the gene. Specific examples of the nucleic acid amplification include quantitative PCR such as quantitative real-time PCR.

In a described diagnostic method, whether or not stage I lung adenocarcinoma is poor-prognosis is determined by examining an enhanced expression of α-actinin-4 protein in a stage I lung adenocarcinoma tissue or cell, whereby poor-prognosis stage I lung adenocarcinoma is detected. The above method includes a step of measuring an expression level of ACTN4 gene in the aforementioned stage I lung adenocarcinoma tissue or cell. When the expression level is larger than that in a corresponding normal tissue or cell, that is, an expression of α-actinin-4 protein is enhanced, the stage I lung adenocarcinoma in a subject mammal is determined (diagnosed) as poor-prognosis stage I lung adenocarcinoma.

A publicly known, standardized method in the art can be used to measure an expression level of ACTN4 gene. An expression level of general gene is typically measured using an mRNA or protein level as an index. Similarly in the present invention the use of a kit is claimed, wherein an expression level of α-actinin-4 protein can be measured by using an mRNA or protein level of ACTN4. The ACTN4 gene sequence and the sequence of an amino acid encoded by the gene are as described above, and a person skilled in the art can measure the expression level of α-actinin-4 protein using a standardized method in reference to these sequences.

According to a preferred embodiment of the present invention drawn to the use of a kit includes, a step of measuring an expression level of a gene includes measuring an expression level of α-actinin-4 protein in the aforementioned tissue or cell. The expression level of α-actinin-4 protein can be measured by a method known in the art. For example, the amino acid sequence shown in SEQ ID NO: 2 may be given as an amino acid sequence of α-actinin-4 protein, and a person skilled in the art can appropriately measure the amount of the protein in reference to the sequence.

The amount of α-actinin-4 protein can be preferably measured by using, for example, an antibody that binds to the protein. Such an antibody is preferably an antibody specific to α-actinin-4 protein. The antibody may be a polyclonal antibody or a monoclonal antibody. Determination of α-actinin-4 protein is preferably performed by immunohistochemical staining using such an antibody. Alternatively, an immunoblot assay, in which protein extracted from the aforementioned tissue or cell will be subjected to electrophoresis and then determined using an antibody, and the like can be used.

According to another preferred embodiment of the present invention the claimed use includes, a step of measuring an expression level of gene includes determining ACTN4 mRNA in the aforementioned tissue or cell. For example, the nucleotide sequence shown in SEQ ID NO: 1 (wherein, t in the sequence is converted to u) may be given as ACTN4 mRNA, and a person skilled in the art can appropriately determine ACTN4 mRNA in reference to the sequence. Specific examples of determination methods include a method in which amplification is performed by RT-PCR using a specific primer pair, followed by electrophoresis.

A normal tissue or cell to be used as a comparative control may be a normal tissue or cell obtained from the identical subject individual or that obtained from a different individual. Also, expression levels of α-actinin-4 protein in normal tissues and cells of a plurality of different individuals are measured and an average value thereof can be used as a comparative control. Further, an appropriate value such as an average value + standard deviation is set in advance based on data of expression levels in a plurality of normal tissues and cells measured in advance, and when the expression level in the test tissue or cell is higher than the appropriate value, it can be determined that the expression of ACTN4 gene is enhanced. Examples of the "corresponding normal tissue or cell" include a normal lung tissue or cell.

In the present specification, it is verified that when a serum CEA concentration of a subject mammal is increased along with an enhanced α-actinin-4 protein expression, the stage I lung adenocarcinoma developed in the subject can be determined as poor-prognosis stage I lung adenocarcinoma with further remarkable significant difference. Therefore a diagnostic method is described, which includes the following steps of; (c) measuring a concentration of carcinoembryonic antigen (CEA) in serum of the aforementioned mammal, and further, (d) comparing the concentration obtained in (c) with a concentration of carcinoembryonic antigen (CEA) in normal serum. According to the above diagnostic method, when the expression of α-actinin-4 protein in the aforementioned stage I lung adenocarcinoma tissue or cell is enhanced compared to that in the normal tissue or cell, and the concentration of carcinoembryonic antigen (CEA) in the serum of the aforementioned mammal is significantly higher than that in the aforementioned normal serum, it is indicated that the stage I lung adenocarcinoma developed in the mammal is poor-prognosis stage I lung adenocarcinoma.

A publicly known, standardized method in the art can be used as a method for measuring a serum concentration of CEA. CEA is a well-known tumor marker, and various methods for measuring a serum concentration of CEA are known such as ELISA and CLEIA, and a kit for carrying out each of the above methods is commercially available. Normal serum to be used as a comparative example may be normal serum obtained from the identical subject individual, or that obtained from a different individual. Also, concentrations of CEA in normal serum of a plurality of different individuals are measured and an average value thereof can be used as a comparative control. Further, an appropriate value such as an average value + standard deviation is set in advance based on data of concentrations of CEA in a plurality of normal serum measured in advance, and when the concentration in the test serum is higher than the appropriate value, it can be determined that the serum concentration of CEA is increased. For example, in human serum, when a concentration of CEA exceeds 5.0 ng/mL, it can be determined that the concentration is significantly higher than that in normal serum.

Described is a further diagnostic method performed in combination with the above described diagnostic method. That is, this described diagnostic method further includes the following step of: (e) measuring the copy number of ACTN4 gene in the genome in a stage I lung adenocarcinoma tissue cell obtained from the aforementioned mammal. According to the above method, when an expression level of α-actinin-4 protein in the aforementioned stage I lung adenocarcinoma tissue or cell is enhanced compared to that in the aforementioned normal tissue or cell, and the copy number of ACTN4 gene exceeds 4, it is indicated that the stage I lung adenocarcinoma developed in the aforementioned mammal is poor-prognosis stage I lung adenocarcinoma.

In order to practice the method of detection/diagnosis necessary reagents can be collectively provided as a kit. Therefore, according to the present invention, the use of a kit for in vitro detecting poor-prognosis stage I lung adenocarcinoma in a mammal is provided, and the kit includes a reagent capable of measuring the copy number of ACTN4 in the genome in a cell. Such a reagent is selected depending on a specific method to be practiced, and it is preferably a probe specific to the gene in the genome that can be used for FISH. The aforementioned specific probe preferably consists of the nucleotide sequence of the region from 43.81 Mb to 44.02 Mb on human chromosome 19.

The use of a kit according to another embodiment of the present invention is the use for in vitro detecting poor-prognosis stage I lung adenocarcinoma in a mammal, including a reagent capable of measuring expression levels of ACTN4 gene and α-actinin-4 protein in a tissue or cell. As such a reagent, a reagent to determine α-actinin-4 protein in the aforementioned tissue or cell, for example, an antibody specifically binding to α-actinin-4 protein, can be preferably used. Alternatively, such a reagent may also be a reagent to determine ACTN4 mRNA in the aforementioned tissue or cell, for example, a specific primer used for RT-PCR using ACTN4 mRNA as a template and another reagent for RT-PCR. In a preferred embodiment of the claimed use, the kit further includes a reagent capable of measuring a serum concentration of carcinoembryonic antigen (CEA). In another preferred embodiment of the claimed use, the kit further includes a reagent capable of measuring the copy number of ACTN4 gene. Such a reagent is preferably a probe specific to the gene in the genome that can be used for FISH. The aforementioned specific probe preferably consists of the nucleotide sequence of the region from 43.81 Mb to 44.02 Mb on human chromosome 19.

The kit used in present invention may further include other reagents, a reaction container, an instruction, and the like, according to a specific method to be practiced.

Described is also a method for treating or preventing stage I lung adenocarcinoma, including administering to a subject a therapeutically effective amount of an ACTN4 inhibitor, is provided. Further described is the use of an ACTN4 inhibitor for the production of a medicine for treating stage I lung adenocarcinoma is provided.

A subject to whom treatment or prevention is given is preferably a mammal, for example, a human or a non-human mammal.

It is also described that the ACTN4 inhibitor can be a small molecule such as a medicine. Such a small molecule is obtainable through screening by a publicly known method in the art, using an ACTN4-inhibitory activity as an index.

Moreover described is that the ACTN4 inhibitor is an antibody specifically binding to α-actinin-4 protein. The antibody can be either a polyclonal antibody or a monoclonal antibody; however, it is preferably a monoclonal antibody.

Described is also that the ACTN4 inhibitor can be an antisense nucleic acid molecule specifically inhibiting an expression of ACTN4 gene. An antisense method is a known technique to inhibit a specific gene expression. In a specific example, the aforementioned antisense nucleic acid molecule is an approximately 10- to 40-base-long antisense RNA designed based on a sequence of the 5' coding region of ACTN4 gene. In another specific example, the aforementioned antisense nucleic acid molecule is a DNA oligonucleotide designed to be complementary to a sequence of a region involved in the transcription of the ACTN4 gene. Such antisense nuclei acid molecules can be easily designed based on the ACTN4 gene sequence as shown in SEQ ID NO: 1.

It is also described that the ACTN4 inhibitor can be a siRNA nucleotide molecule specifically inhibiting an expression of ACTN4 gene. In the present specification, the "siRNA nucleotide molecule" means not only siRNA per se but also a longer double-stranded RNA molecule capable of introducing siRNA into a target cell. The siRNA nucleotide molecule is a known tool capable of inhibiting a specific gene expression by RNA interfering (RNAi) (Elbashir, S.M. et al., Nature 411, 494-498, 2001). Typically, siRNA consists of a nucleotide sequence containing 19 to 21 base pairs homologous to a sequence specific to target gene mRNA. The aforementioned double-stranded RNA molecule typically consists of a longer nucleotide sequence homologous to a sequence specific to target gene mRNA. Such siRNA can be easily designed based on the ACTN4 gene sequence as shown in SEQ ID NO: 1. Further, the aforementioned siRNA nucleotide molecule can also be expressed by an appropriate vector delivered into a cell. Accordingly, the ACTN4 inhibitor may also be a vector expressing a siRNA nucleotide molecule that specifically inhibits an expression of ACTN4 gene. Such a vector can be easily constructed according to a standardized procedure known in the art. (Bass, B.L., Cell 101, 235-238, 2000; Tavernarakis, N. et al., Nat. Genet. 24, 180-183, 2000; Malagon, F. et al., Mol. Gen. Genet. 259, 639-644, 1998; Parrish, S. et al., Mol. Cell 6, 1077-1087, 2000).

The ACTN4 inhibitor can be administered via a suitable route, for example, locally, intravenously, subcutaneously, intramuscularly, orally, or through rectum or mucous membrane. The therapeutically effective dosage is determined properly by a doctor or veterinarian according to severity of symptoms, age of the patient, efficacy of the specific drug used, administration route, frequency of administration and the like. Generally, the therapeutically effective dosage is approximately 0.001 to approximately 1000 mg/kg body weight, preferably approximately 0.01 to approximately 10 mg/kg body weight, more preferably approximately 0.01 to approximately 1 mg/kg body weight per day.

The ACTN4 inhibitor may be administered together with a pharmaceutically allowable carrier. Thus, the present specification also describes a pharmaceutical composition for treatment of stage I lung adenocarcinoma, comprising an ACTN4 inhibitor and a pharmaceutically allowable carrier.

The pharmaceutically allowable carrier, such as vehicle, diluent base or diluent, is selected properly by those who are skilled in the art according to the administration route and the properties of the specific drug used.

The present specification also describes a method for treating stage I lung adenocarcinoma, including a step of administering a therapeutically effective amount of a chemotherapeutic agent to patients with stage I lung adenocarcinoma, who are determined to have poor-prognosis by the diagnostic method in the first aspect or the diagnostic method in the second aspect of the present invention, or by a combination thereof. The chemotherapeutic agent may be the ACTN4 inhibitor described above, a tegafur-5-fluorouracil preparation, another anticancer agent such as cisplatin or the like.

### EXAMPLE

Hereinafter, the present invention will be described more specifically with reference to Examples, which should be understood that the present invention is not restricted thereby.

### Example 1: Relationship between stage I lung adenocarcinoma and acceleration of α-actinin-4 gene expression

The samples used were formalin-fixed paraffin-embedded resected samples from 372 patients, who were subjected to excision surgery of stage I lung adenocarcinoma in the Hospital of National Cancer Center (Japan) during the period from Dec. 1997 to March 2001 (hereinafter, referred to as "cohort 1"). Expression of α-actinin-4 protein in these samples was evaluated by immunohistochemical assay.

The primary antibody used in the immunohistochemical assay was anti-human α-actinin-4 rabbit polyclonal antibody prepared in the Research Institute of National Cancer Center (Japan). Immunostaining was carried out by ABC method of using an avidin-biotin complex.

In evaluation of the expression of α-actinin-4 protein, samples in which the expression thereof was accelerated in the cancer region than in the vascular endothelial cells in the same section and the expression-accelerated region occupied 30% or more of the entire tumor area were defined to be positive, and the other sample to be negative.

Then, the classification of α-actinin-4 protein expression and the risk of various clinical pathological factors on the lifetime of patients with stage I lung adenocarcinoma were analyzed by monovariate and multivariate analyses in Cox proportional hazard model. The variables used were, in addition to α-actinin-4 protein expression (positive /negative), sex (male/female), age (65 or older/younger than 65), smoking status (no/yes), stage (IA/IB), serum CEA concentration (more than 5 ng/mL/5 ng/mL or less), lymph duct invasion (positive /negative) and vein invasion (positive/negative). The results are summarized in the following Table 1.

**[Table 1]**

| Analysis of the factors having an influence on clinical pathological outcome of the patients with stage I lung adenocarcinoma by using Cox proportional hazard model (cohort 1: analysis of α-actinin-4 protein expression) | | | | | | | |
|---|---|---|---|---|---|---|---|
| | | Monovariate analysis | | | Multivariate analysis | | |
| Variable | N | Hazard rate | 95% Confidence interval | P | Hazard rate | 95% Confidence interval | P |
| Sex (male/female) | 182/190 | 2.1011 | 1.2388 - 3.5634 | 0.00588 | 1.581 | 0.8484 - 2.9462 | 0.149198 |
| | | | | | | | |
| Age (65 or older/younger than 65) | 200f 172 | 2.5009 | 1.9286 - 4.3783 | 0.00134 | 2.3074 | 1.3012 - 4.0916 | 0.00422 |
| | | | | | | | |
| Smoking status (no/yes) | 165/207 | 2.3957 | 1.4203 - 4.0410 | 0.00106 | 1. 7603 | 0.9562 - 3.2403 | 0.069338 |
| | | | | | | | |
| Stage (IA/IB) | 295/77 | 2.6628 | 1.5773 - 4.4953 | 0.00025 | 1.9322 | 1.1150 - 3.3483 | 0.01887 |
| | | | | | | | |
| Serum CEA (>5ng/mL / 5ng/mL≧) | 62/310 | 3.0656 | 1.8055 - 5.2049 | 3.4E-05 | 2.1566 | 1.2491 - 3.7237 | 0.00581 |
| | | | | | | | |
| Lymph duct invasion (positive/negative) | 199/173 | 2.8862 | 1.7313 - 4.8082 | 4.8E-05 | 2.5613 | 1.5066 - 1.3646 | 0.00052 |
| | | | | | | | |
| Vein invasion (positive/negative) | 106/263 | 2.5171 | 1.5221 - 4.1624 | 0.00032 | 1.4718 | 0.8629 - 2.5105 | 0.156032 |
| | | | | | | | |
| α-Actinin-4 protein expression (positive/negative) | 199/173 | 2.1146 | 1. 2196 - 3.6664 | 0.00765 | 2.1153 | 1.1955 - 3.7425 | 0.01007 |

The results of monovariate analysis summarized in the left side of Table 1 showed that all of these items could be risk factors which lead to a poor-prognosis.

In addition, the right side of Table 1 summarizes the results obtained by multivariate analysis. The results indicated that age, stage, serum CEA concentration, lymph duct invasion and α-actinin-4 protein expression showed a statistically significant difference, indicating that these items were the risk factors which lead to a poor prognosis. Thus, an intensity of the α-actinin-4 protein expression in stage I lung adenocarcinoma cells was shown to prognosis factor independent of the other clinical pathological factors studied here.

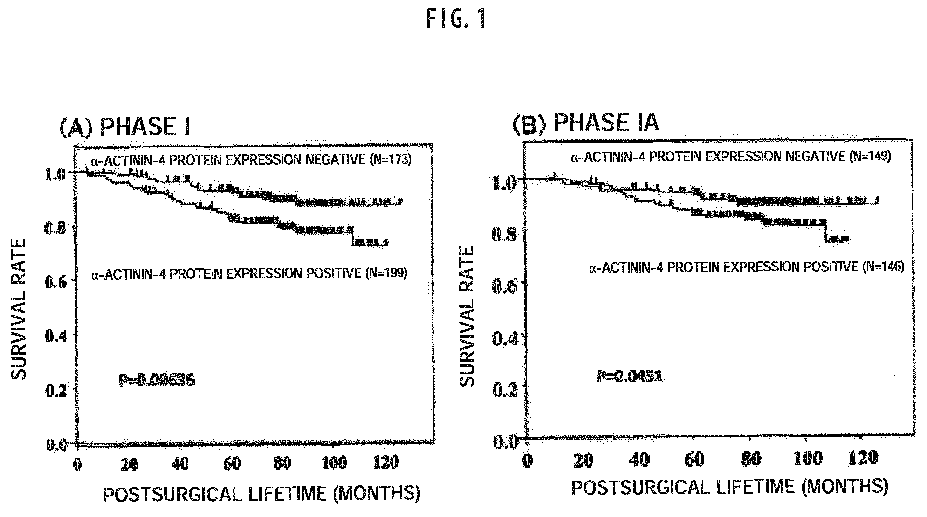

Survival curves of the two groups of incidences, positive and negative cases of α-actinin-4 protein expression, classified as described above were then drawn by the Kaplan-Meier method and examined by the Log-rank test. The survival curves obtained were shown in Figure 1. Panel A of Figure 1 shows survival curves of an entire stage I. The 5-year survival rate of the entire stage I in the negative cases was 92.7%, while the 5-year survival rate stage in the positive cases was 82.1%. Panel B of Figure 1 shows survival curves only of stage IA. The 5-year survival rate of the stage IA in the negative cases was 94.4%, while the 5-year survival rate thereof in the positive cases was 86.5%. According to both panels A and B, the difference in survival rate between the negative and positive cases increases after 5 years from surgery. Thus, Figure 1 shows that the positive cases of α-actinin-4 protein expression have a poorer prognosis than that of the negative cases with a statistically significant difference.

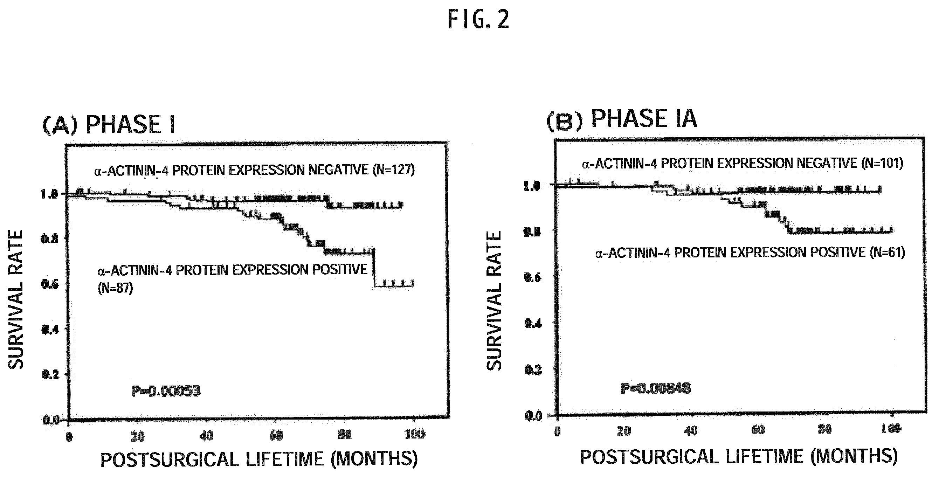

A similar study was also made by using formalin-fixed paraffin-embedded resected samples from 214 patients, who were subjected to excision surgery of stage I lung adenocarcinoma in the Hospital of National Cancer Center (Japan) during the period from March 2001 to Jan. 2003 (hereinafter, referred to as "cohort 2"). The results are summarized in Table 2 and Figure 2.

**[Table 2]**

| Clinical-pathological analysis of prognostic factors in stage I lung adenocarcinoma by using Cox proportional hazard model (cohort 2: Analysis of α-actinin-4 protein expression) | | | | | | | |
|---|---|---|---|---|---|---|---|
| | | Monovariate analysis | | | Multivariate analysis | | |
| Variable | N | Hazard rate | 95% Confidence interval | P | Hazard rate | 95% Confidence interval | P |
| Sex (male/female) | 100/114 | 0.8792 | 0.3990 - 1.9376 | 0.749496 | | | |
| | | | | | | | |
| Age (65 or older/younger than 65) | 115/99 | 1.8251 | 0.8177 - 4. 0736 | 0.14192 | | | |
| | | | | | | | |
| Smoking status (no/yes) | 120/94 | 0.7797 | 0.3437 - 1.7690 | 0.55164 | | | |
| | | | | | | | |
| Stage (IA/IB) | 162/52 | 2.1994 | 0.9871 - 4.9004 | 0.053825 | | | |
| | | | | | | | |
| Serum CEA (>5ng/mL / 5ng/mL≧) | 36/178 | 4.2755 | 1.9096 - 9.5731 | 0.00041 | 3.4228 | 1.4977 - 7.8222 | 0.00325 |
| | | | | | | | |
| Lymph duct invasion (positive/negative) | 57/157 | 3.8748 | 1.7566 - 8.5475 | 0.00079 | 1.9355 | 0.7042 - 5.3194 | 0.200464 |
| | | | | | | | |
| Vein invasion (positive/nagative) | 63/161 | 3. 2821 | 1.4871 - 7.2441 | 0. 00326 | 1.7957 | 4.6536 - 4.9336 | 0.256259 |
| | | | | | | | |
| α-Actinin-4 protein expression (positive/negative) | 87/127 | 4.4238 | 1.7654 - 11.0849 | 0.0151 | 3.5239 | 1.3904 - 8.9312 | 0.00794 |

The results obtained by monovariate analysis shown in the left side of Table 2 showed that serum CEA, lymph duct invasion, vein invasion and α-actinin-4 protein expression could be the risk factors which lead to a poor prognosis.

In addition, the right side of Table 2 shows the results obtained by multivariate analysis only of the serum CEA, lymph duct invasion, vein invasion and α-actinin-4 protein expression, which were shown to be factors that lead to a poor prognosis by monovariate analysis. The results showed that serum CEA and α-actinin-4 protein expression showed a statistically significant difference, indicating that these items could be risk factors that lead to a poor prognosis. Thus, the intensity of the α-actinin-4 protein expression in stage I lung adenocarcinoma cells are prognostic factors independent of other clinical pathological factors studied here.

Panel A of Figure 2 shows survival curves of an entire stage I. The 5-year survival rate of the negative cases in stage I was 95.9%, while the 5-year survival rate of the positive cases in stage I was 87.9%. Panel B of Figure 2 shows survival curves only of the stage IA. The 5-year survival rate of the negative cases in the stage IA was 95.9%, while the 5-year survival rate of the positive cases in the stage IA was 89.7%. According to both panels A and B, the difference in survival rate between the negative and positive cases increases after 5 years from surgery. Thus, Figure 2 shows that the positive cases of α-actinin-4 protein expression have a poorer prognosis than the negative cases of α-actinin-4 protein expression with a statistically significant difference.

### Example 2: Prediction of prognosis for stage I lung adenocarcinoma by a combination of acceleration of α-actinin-4 protein expression and serum CEA concentration

The relevance of the combination of acceleration of α-actinin-4 protein expression and serum CEA concentration to a prognosis in the stage I lung adenocarcinoma were studied for the cohorts 1 and 2 described in Example 1. Specifically, the cohorts 1 and 2 were divided into the following three groups: a group of serum CEA of 5 ng/mL or less with negative α-actinin-4 protein; a group of serum CEA of more than 5 ng/mL or α-actinin-4 protein positive; and a group of serum CEA more than 5 ng/mL with positive α-actinin-4 protein, and survival curves of each group were drawn by the Kaplan-Meier method and analyzed by the Log-rank test.

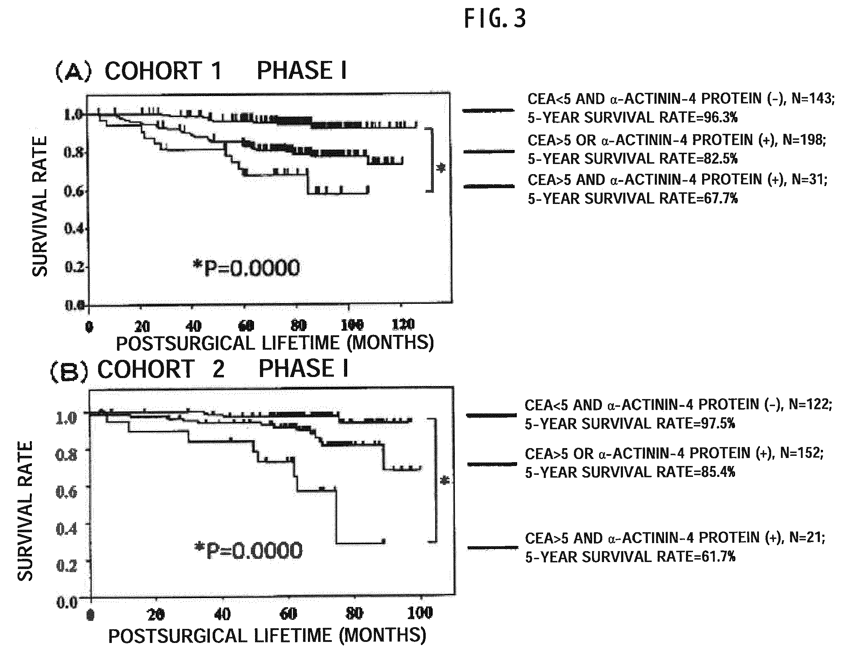

The survival curves obtained are shown in Figure 3. Figure 3 shows that the combination of acceleration of α-actinin-4 protein expression and serum CEA concentration is a severer risk factor that lead to a poor prognosis, indicating that the accelerated of α-actinin-4 protein expression in stage I lung adenocarcinoma cells and a serum CEA concentration of more than 5 ng/mL have a distinctively poor postoperative prognosis.

### Example 3: Increase in genome copy number of ACTN4 gene in stage I lung adenocarcinoma

A probe specific to the ACTN4 gene present on the long arm of chromosome 19 was prepared to study the copy number of ACTN4 gene on genome, and paraffin sections of dissected stage I lung adenocarcinoma samples were analyzed by the FISH (Fluorescence in Situ Hybridization) method.

The copy number of ACTN4 gene in the paraffin sections of the cohort 1 described in Example 1 was determined by the FISH method. PathVysion DNA probe kit (Abbott Molecular, Des Plaines, IL) was used in measurement by the FISH method. The specific probe used in hybridization was a denatured bacterial artificial chromosome (BAC) probe (red) containing the region from 43.81 Mb to 44.02 Mb on human chromosome 19 including ACTN4 (RP11-118P21) gene locus. Chromosome 19 centromere control clone (green) was also used. These probes were labeled by using SpectrimOrange (Abbott Molecular, Des Plaines, IL). Hybridization was carried out at 37°C for 14 to 18 hours. Red and green fluorescence signals in 20 cell nuclei were counted according to the method of Herceptin test, and samples having a value obtained by dividing the red signal count by the green signal count of more than 2 were defined to be ACTN4 amplification (+) (FISH positive) and those having a count of 2 or less to be ACTN4 amplification (-) (FISH negative).

Then, the classification on ACTN4 gene amplification and the risk of various clinical pathological factors on the survival time of stage I lung adenocarcinoma were analyzed by monovariate and multivariate analyses in the Cox proportional hazard model. The variables used were, in addition to the ACTN4 amplification (positive /negative), sex (male/female), age (65 or older/younger than 65), smoking status(no/yes), stage (IA/IB), serum CEA concentration (more than 5 ng/mL/5 ng/mL or less), lymph duct invasion (positive /negative) and vein invasion (positive/negative). The results are summarized in the following Table 3.

**[Table 3]**

| Clinical-pathological analysis of prognostic factors for stagestage I lung adenocarcinoma by using Cox proportional hazard model (cohort 1: analysis of ACTN4 gene copy number | | | | | | | |
|---|---|---|---|---|---|---|---|
| | | Monovariate analysis | | | Multivariate analysis | | |
| Variable | N | Hazard rate | 95% Confidence interval | P | Hazard rate | 95% Confidence interval | P |
| Sex (male/female) | 98/10B | 2.3951 | 1.1277 - 5.0868 | 0.02304 | 2.4325 | 1.1437 - 5.1738 | 0.02097 |
| | | | | | | | |
| Age (65 or older/younger than 65) | 86/120 | 2.2737 | 1. 0167 - 5.0848 | 0.04698 | 2.7561 | 1.2133 - 6.2635 | 0.01545 |
| | | | | | | | |
| Smoking status (no/yes) | 123/83 | 1.8802 | 0.9266 - 3.8152 | 0.084346 | | | |
| | | | | | | | |
| Stage (IA/IB) | 154/52 | 2.3146 | 1.1227 - 4.7718 | 0.023 | 2.5259 | 1. 1998 - 5.3179 | 0.01471 |
| | | | | | | | |
| Serum CEA (>5ng/mL / 5ng/mL≧) | 34/172 | 1.8416 | 0.8239 - 4. 1189 | 0.137092 | | | |
| | | | | | | | |
| Lymph duct invasion (positive/negative) | 36/170 | 2.5771 | 1.2117 - 5.4808 | 0.01394 | 2.8193 | 1.2814 - 6.2026 | 0.00998 |
| | | | | | | | |
| Vein invasion (positive/negative) | 64/142 | 2.024 | 0.9973 - 4.1079 | 0.050897 | | | |
| | | | | | | | |
| ACTN4 gene amplification (positive/negative) | 20/186 | 4.2287 | 1.9445 - 9.1960 | 0.00028 | 3.9933 | 1.7878 - 8.9195 | 0.00073 |

The results of monovariate analysis shown in the left side of Table 3 show that sex, age, stage, lymph duct invasion and ACTN4 amplification could be the risk factors which lead to a poor prognosis.

The right side of Table 3 shows the results obtained by multivariate analysis only of sex, age, stage, lymph duct invasion and ACTN4 amplification which were found to be the potential factors that lead to a poor prognosis in the monovariate analysis above. The results showed that sex, age, stage, lymph duct invasion and ACTN4 amplification showed a statistically significant difference, indicating that these items were risk factors which lead to a poor prognosis. It was thus shown that the ACTN4 amplification in stage I lung adenocarcinoma cells was a prognostic factor independent of the other clinical pathological factors studied here.

### Example 4: Prediction of prognosis for stage I lung adenocarcinoma by a combination of acceleration of α-actinin-4 protein expression and ACTN4 gene amplification

The relevance of the combination of acceleration of α-actinin-4 protein expression and ACTN4 gene amplification to the prognosis in stage I lung adenocarcinoma was studied in the cohort 1 described in Example 1. Specifically, the samples were divided into the following three groups: a group of ACTN4 gene amplification (-) and α-actinin-4 protein expression negative; a group of ACTN4 amplification (-) and actinin-4 expression positive; and a group of ACTN4 gene amplification (+) and α-actinin-4 protein expression positive, and a survival curve of each group was drawn by the Kaplan-Meier method and analyzed by the Log-rank test.

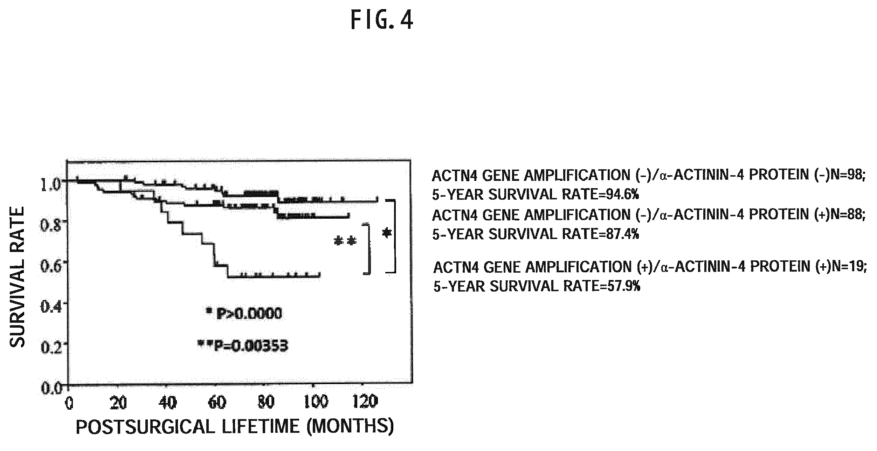

The survival curves obtained were shown in Figure 4. Figure 4 shows that the combination of acceleration of α-actinin-4 protein expression and ACTN4 gene amplification is a severer risk factor that lead to a poor prognosis than that of single factor thereof and thus, that patients who exhibit acceleration of α-actinin-4 protein expression and ACTN4 gene amplification in stage I lung adenocarcinoma cells have distinctively poor postoperative prognosis.

### Example 5: Relationship between lung adenocarcinoma and acceleration of α-actinin-4 gene expression

A similar study to Example 1 was made and expression of α-actinin-4 protein was evaluated by immunohistochemical assay. The samples used were formalin-fixed paraffin-embedded resected samples (total 540 = 372 + 168) from 168 patients, who were subjected to excision surgery of stages II and III lung adenocarcinoma in the Hospital of National Cancer Center (Japan) from 1997 to 2001, in addition to cohort 1 in Example 1 (hereinafter, referred to as "cohort 1 "). In addition to cohort 2 described in Example 1, formalin-fixed paraffin-embedded resected samples (total 661 = 214 + 447) from 447 patients, who were subjected to excision surgery of stage I lung adenocarcinoma in the Hospital of National Cancer Center East (Japan) from 1993 to 2005 were used (hereinafter, referred to as "cohort 2"). The definitions of the primary antibody used in the immunohistochemical assay, immunostaining method, positive (IHC-positive) and negative (IHC-negative) are the same as those in Example 1.

An expression of α-actinin-4 protein was analyzed, similar to Example 1, by monovariate analysis and multivariate analysis in Cox proportional hazard model with groups by sex and age, etc., and the hazard rate of each clinical pathological factor on lifetime of patients with lung adenocarcinoma. The results are summarized in the following Table 4.

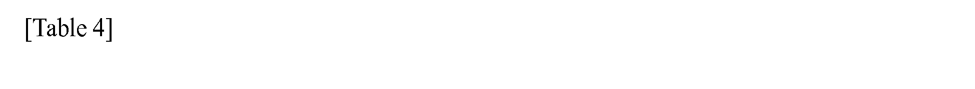

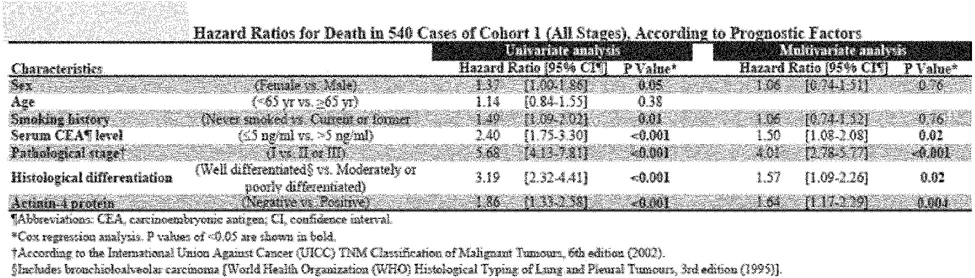

The results of monovariate analysis summarized in the left side of Table 4 show that all of these items above could be risk factors which lead to a poor-prognosis.

Further, the right side of Table 4 summarizes the results obtained by the multivariate analysis. The results indicate that stage, serum CEA concentration, and α-actinin-4 protein expression showed a statistically significant difference, indicating that these items were the risk factors which lead to a poor prognosis. Thus, the intensity of the α-actinin-4 protein expression in stages I-III lung adenocarcinoma cells was shown to be a prognostic factor independent of the other clinical pathological factors studied here.

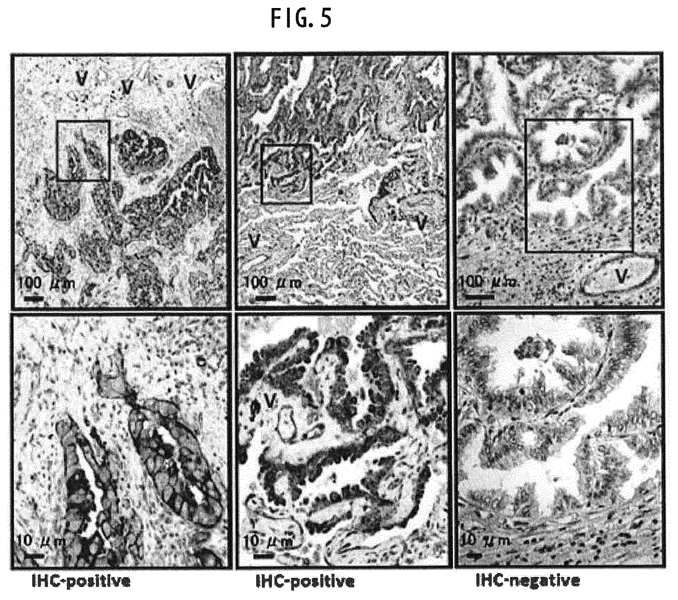

FIG. 5 shows photo-micrographs of lung adenocarcinoma tissue from the two groups divided as above, the positive cases and the negative cases of α-actinin-4 protein expression. The left side and in the middle of FIG. 5 show the results for the positive cases (IHC-positive), while the right side in FIG. 5 shows the results for the negative cases (IHC-negative). The photo-micrographs in the lower side of FIG. 5 show magnified within-frame photos of photo-micrographs that are shown in the upper side thereof. As shown in the figure, actinin-4 protein ("V" in the figure) was extended along alveolar septum, and was localized in cell membrane and cytoplasm of cancer cells invading through interfibrillar mass.

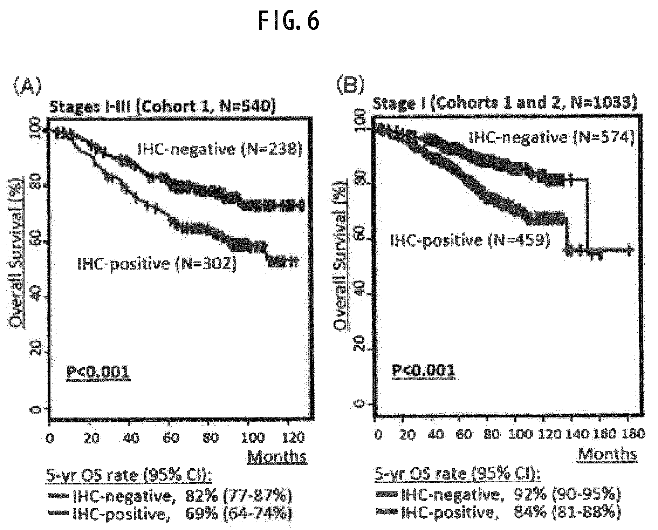

Next, the survival curves for the two groups, the positive cases and the negative cases of α-actinin-4 protein expression were prepared by the Kaplan-Meier method and examined by the Log-rank test. The survival curves obtained are shown in FIG. 6. Panel A of FIG. 6 shows survival curves in stages I-III. The 5-year survival rate of the negative cases in the stages I-III was 82%, while the 5-year survival rate of the positive cases was 69%. Panel B of FIG. 6 shows the survival curves in stage I. The 5-year survival rate of the negative cases in the stage I was 92%, while the 5-year survival rate of the positive cases was 84%. According to both panels A and B, the differences in the survival rates between the negative and positive cases increases after 5 years from surgery. Thus, FIG. 6 shows that the positive cases of α-actinin-4 protein expression have a poorer prognosis than that of the negative cases with a statistically significant difference.

### Example 6: Increase in genome copy number of ACTN4 gene in patients with stage I lung adenocarcinoma

A probe specific to the ACTN4 gene present on the long arm of chromosome 19 was prepared to study the copy number of ACTN4 gene on genome, and paraffin sections of dissected stage I lung adenocarcinoma samples were analyzed using a FISH (Fluorescence in Situ Hybridization) method, similar to that of Example 3. The samples used were formalin-fixed paraffin-embedded resected samples from 543 patients, who were subjected to excision surgery of stages I-IV lung adenocarcinoma in the Hospital of National Cancer Center (Japan) from 1997 to 2009. Of 543 samples, fibroblast growth factor receptor (FGFR) gene mutation was present in 288 samples, while no mutation was present in 255 samples. These 543 samples do not overlap with cohorts 1 and 2 described in Example 5. Similar definitions to those used in Example 3 for the probe specific, positive (FISH-positive) and negative (FISH-negative), are used.

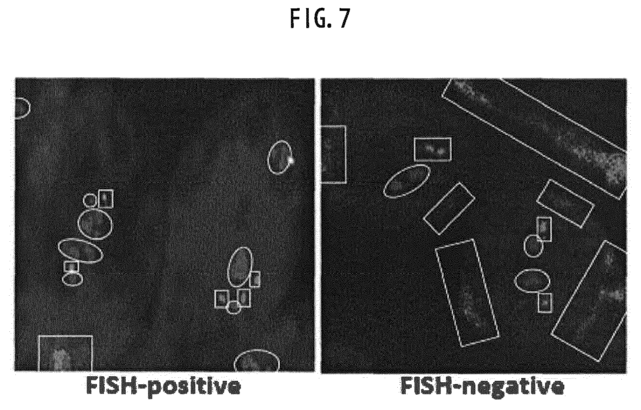

FIG. 7 shows photo-micrographs of lung adenocarcinoma cells signaled by the Fluorescence in Situ Hybridization (FISH) method. The left side in FIG. 7 shows the results for positive cases (FISH-positive), while the right side in FIG. 7 shows the results for negative cases (FISH-negative). As shown in the figure, red (o in FIG. 7) and green (□ in FIG. 7) fluorescence signals (fluorescence signals) in lung adenocarcinoma cells were acquired. The FISH positive 79 resected samples (15%) out of 543 resected samples in stages I-IV were detected according to the definitions of FISH positive and negative.

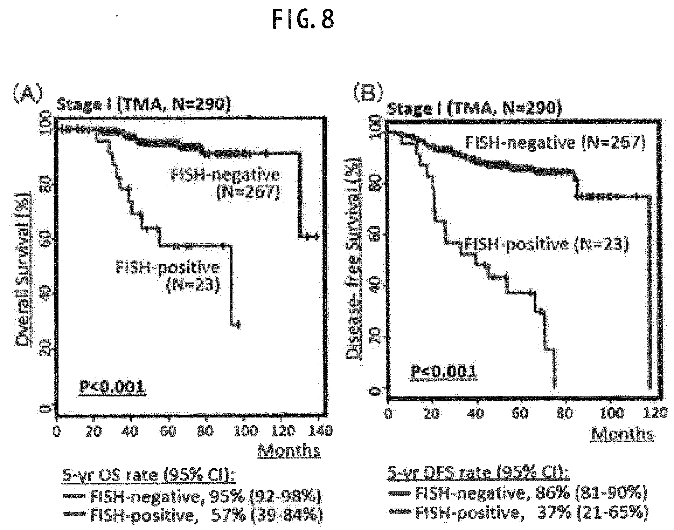

Further, survival curves were drawn by the Kaplan-Meier method for the two groups divided by the FISH method, the positive cases and the negative cases, and were analyzed by the Log-rank test. The obtained survival curves are shown in FIG. 8. Panel A of FIG. 8 shows the survival curves in the stage I. The 5-year survival rate of the negative cases in the stage I was 95%, while the 5-year survival rate thereof in the positive cases was 57%. Panel B of FIG. 8 shows the survival curves in the stage I. The 5-year survival rate of the stage I patients in the negative group was 86%, while the 5-year survival rate thereof in the positive cases was 37%. According to the both Panels A and B, the differences in survival rates between the negative and positive cases increase after 5 years from surgery. Thus, FIG. 8 shows that the positive cases of α-actinin-4 protein expression have a poorer prognosis than that of the negative cases with a statistically significant difference.

### Example 7: Combination of immunohistochemical assay and FISH method

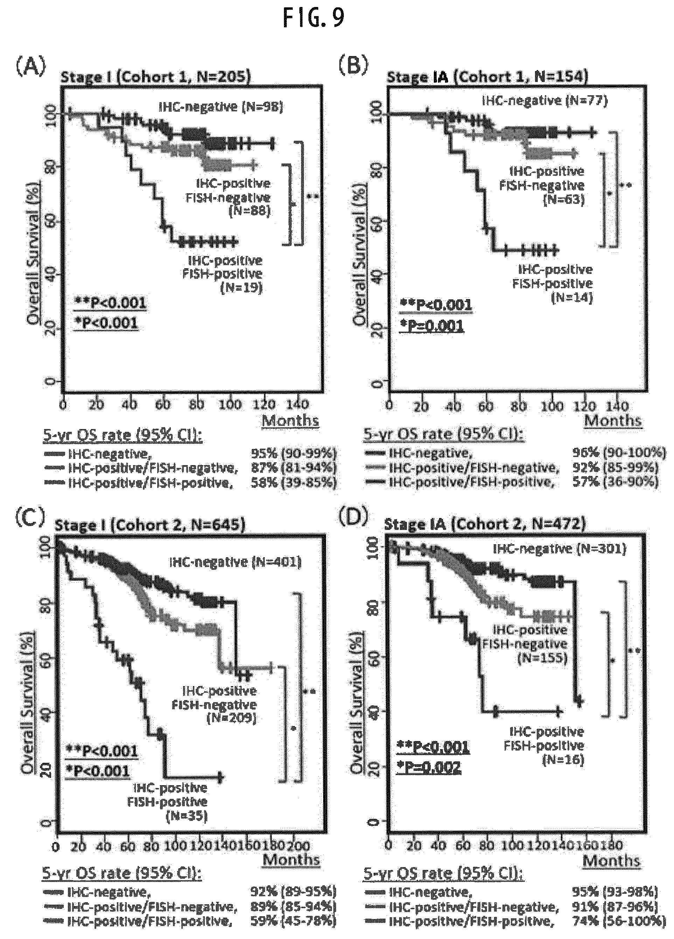

The samples used were 206 resection samples of stage I lung adenocarcinoma from cohort 1 used in Example 5, and 645 resection samples of stage I lung adenocarcinoma from cohort 2 used in Example 5. The negative and positive cases of α-actinin-4 protein expression were combined respectively with the positive and negative cases of the FISH method analysis, and the survival curves of these samples were drawn by the Kaplan-Meier method, then analyzed by the Log-rank test. The obtained survival curves are shown in FIG. 9. Panel A of FIG. 9 shows the survival curves of cohort 1 in the stage I. The 5-year survival rate of the negative cases of α-actinin-4 protein expression in the stage I was 95%, while the positive cases of α-actinin-4 protein expression with the negative cases of the FISH method analysis was 87%. On the other hand, the 5-year survival rate of the positive cases of α-actinin-4 protein expression with the positive cases of the FISH method analysis was 58%. Further, Panel B of FIG. 9 shows the survival curves of cohort 1 in the stage IA. The 5-year survival rate of the negative cases of α-actinin-4 protein expression was 96%, while the positive cases of α-actinin-4 protein expression with the negative cases of the FISH method analysis was 92%. On the other hand, the 5-year survival rate of the positive cases of α-actinin-4 protein expression and with the positive cases of the FISH method analysis was 57%. Thus, Panels A and B show that the positive/negative cases of the FISH method analysis have a poorer prognosis than that of the positive/negative cases of α-actinin-4 protein expression with a statistically significant difference.

Further, Panel C of FIG. 9 shows the survival curves of cohort 2 in the stage I. The 5-year survival rate of the negative cases of α-actinin-4 protein expression was 92%, while the positive cases of α-actinin-4 protein expression with the negative cases of the FISH method analysis was 89%. On the other hand, the 5-year survival rate of the positive cases of α-actinin-4 protein expression with the positive cases of the FISH method analysis was 59%. Further, Panel D of FIG. 9 shows the survival curves of cohort 2 in the stage IA. The 5-year survival rate of the negative cases of α-actinin-4 protein expression was 95%, while the 5-year survival rate of the positive cases of α-actinin-4 protein expression with the negative cases of α-actinin-4 protein expression was 91%. On the other hand, the 5-year survival rate of the positive cases of α-actinin-4 protein expression with the positive cases of the FISH method analysis was 74%. Thus, Panels C and D show that the positive/negative cases have a poorer prognosis than that of the positive/negative cases of α-actinin-4 protein expression with a statistically significant difference.

It should be understood that the present invention is not intended to be limited to the modifications and the preferred embodiments contained herein. Various changes and modifications apparent to those skilled in the art are deemed to be within the spirit and scope of the present invention.

This application is based on Japanese Patent Application No. 2010-78772, filed on March 30, 2010, and incorporates the specification, scope of claims, and drawings thereof. The entire disclosure of the above Japanese Patent Application is incorporated herein by reference.

### Industrial Applicability

The present invention, the use of a kit, comprising either
- a probe that is available for determining the copy number of ACTN4 gene in a cell
- or a reagent for quantitative determination of α-actinin-4 protein in the tissue or cell
- can be used for in vitro detecting a poor-prognosis stage I lung adenocarcinoma in a mammal.

## Claims

1. A use of a kit, comprising either
- a probe that is available for determining the copy number of ACTN4 gene in a cell
- or a reagent for quantitative determination of α-actinin-4 protein in the tissue or cell
- for in vitro detecting a poor-prognosis stage I lung adenocarcinoma in a mammal.

2. The use of the kit according to claim 1, wherein the probe is specific for the gene on genome.

3. The use of the kit according to claim 2, wherein the specific probe has the nucleotide sequence of BAC clone RP11-118P21.

4. The use of the kit according to any one of claims 1 to 3, wherein the copy number of ACTN4 gene is determined by a FISH method using the probe specific for the gene on genome.

5. The use of the kit according to any one of claims 1 to 4, wherein when the copy number of the gene exceeds 4, the stage I lung adenocarcinoma in the mammal is predicted to be a poor-prognosis stage I lung adenocarcinoma.

6. The use of the kit according to any one of claims 1 to 5, for further comparing the expression level measured of α-actinin-4 protein in the tissue or cell with the expression level of α-actinin-4 protein in the corresponding normal tissue or cell, **characterized in that**,
when α-actinin-4 protein expression is enhanced greater in the tissue or cell of the stage I lung adenocarcinoma than that of the normal tissue or cell, the stage I lung adenocarcinoma in the mammal is predicted to be a poor-prognosis stage I lung adenocarcinoma.

7. The use of the kit according to claim 1, wherein the reagent for quantitative determination of the α-actinin-4 protein is an antibody specifically binding to the α-actinin-4 protein.

8. The use of the kit according to any one of claims 1 or 7, **characterized in that** the α-actinin-4 protein has the amino acid sequence represented by SEQ ID No. 2.

9. The use of the kit according to any one of claims 1 to 8, further comprising a reagent that can measure the concentration of carcinoembryonic antigen (CEA) in serum.

10. The use of the kit according to claim 9, for further comparing the concentration measured of carcinoembryonic antigen (CEA) in serum with the concentration of the carcinoembryonic antigen (CEA) in normal serum, **characterized in that**,
when α-actinin-4 protein expression is enhanced greater in the tissue or cell of stage I lung adenocarcinoma than that of the normal tissue or cell, and the concentration of the carcinoembryonic antigen (CEA) in the serum obtained from the mammal is statistically significantly higher than that in the normal serum, the stage I lung adenocarcinoma in the mammal is predicted to be a poor-prognosis stage I lung adenocarcinoma.

## Patentansprüche

1. Verwendung eines Kits, umfassend entweder
- eine Sonde, die vorhanden ist, um die Kopienzahl des Gens ACTN4 in einer Zelle zu bestimmen
- oder ein Reagens zur quantitativen Bestimmung des Proteins α-Actinin-4 im Gewebe oder in der Zelle
- zur in vitro-Detektion eines Lungen-Adenokarzinoms im Stadium I mit schlechter Prognose in einem Säugetier.

2. Verwendung des Kits nach Anspruch 1, wobei die Sonde spezifisch für das Gen im Genom ist.

3. Verwendung des Kits nach Anspruch 2, wobei die spezifische Sonde die Nukleotidsequenz des BAC-Klons RP11-118P21 aufweist.

4. Verwendung des Kits nach einem der Ansprüche 1 bis 3, wobei die Kopienzahl des Gens ACTN4 durch ein FISH-Verfahren bestimmt wird, wobei die Probe verwendet wird, die spezifisch für das Gen im Genom ist.

5. Verwendung des Kits nach einem der Ansprüche 1 bis 4, wobei, wenn die Kopienzahl des Gens 4 übersteigt, für das Lungen-Adenokarzinoms im Stadium I in dem Säugetier vorausgesagt wird, dass es sich um ein Lungen-Adenokarzinoms im Stadium I mit schlechter Prognose handelt.

6. Verwendung des Kits nach einem der Ansprüche 1 bis 5 zum weiteren Vergleichen des gemessenen Expressionsniveaus des Proteins α-Actinin-4 im Gewebe oder in der Zelle mit dem Expressionsniveau des Proteins α-Actinin-4 in dem (der) entsprechenden normalen Gewebe oder Zelle, **dadurch gekennzeichnet, dass**
wenn die Expression des Proteins α-Actinin-4 in dem Gewebe oder der Zelle des Lungen-Adenokarzinoms im Stadium I stärker ist als jene in dem (der) normalen Gewebe oder Zelle, für das Lungen-Adenokarzinoms im Stadium I in dem Säugetier vorausgesagt wird, dass es sich um ein Lungen-Adenokarzinoms im Stadium I mit schlechter Prognose handelt.

7. Verwendung des Kits nach Anspruch 1, wobei das Reagens für die quantitative Bestimmung des Proteins α-Actinin-4 ein Antikörper ist, der spezifisch an das Protein α-Actinin-4 bindet.

8. Verwendung des Kits nach einem der Ansprüche 1 oder 7, **dadurch gekennzeichnet, dass** das Protein α-Actinin-4 die Aminosäuresequenz aufweist, die durch SEQ ID Nr. 2 repräsentiert wird.

9. Verwendung des Kits nach einem der Ansprüche 1 bis 8, ferner umfassend ein Reagens, das die Konzentration des carcinoembryonalen Antigens (CEA) im Serum messen kann.

10. Verwendung des Kits nach Anspruch 9 zum weiteren Vergleichen der gemessenen Konzentration des carcinoembryonalen Antigens (CEA) im Serum mit der Konzentration des carcinoembryonalen Antigens (CEA) in normalem Serum, **dadurch gekennzeichnet, dass**
wenn die Expression des Proteins α-Actinin-4 in dem Gewebe oder der Zelle des Lungen-Adenokarzinoms im Stadium I stärker ist als jene in dem (der) normalen Gewebe oder Zelle und die Konzentration des carcinoembryonalen Antigens (CEA) in dem von dem Säugetier gewonnenen Serum statistisch signifikant höher als im normalen Serum ist, für das Lungen-Adenokarzinoms im Stadium I in dem Säugetier vorausgesagt wird, dass es sich um ein Lungen-Adenokarzinom im Stadium I mit schlechter Prognose handelt.

## Revendications

1. Utilisation d'un kit, comprenant
- soit une sonde disponible pour déterminer le nombre de copies du gène ACTN4 dans une cellule,
- soit un réactif destiné à une détermination quantitative de la protéine α-actinine-4 dans le tissu ou la cellule,
pour une détection in vitro d'un adénocarcinome pulmonaire de stade I de pronostic défavorable chez un mammifère.

2. Utilisation du kit selon la revendication 1, dans laquelle la sonde est spécifique au gène dans le génome.

3. Utilisation du kit selon la revendication 2, dans laquelle la sonde spécifique présente la séquence de nucléotides de clone BAC RP11-118P21.

4. Utilisation du kit selon l'une quelconque des revendications 1 à 3, dans laquelle le nombre de copies du gène ACTN4 est déterminé par une méthode d'hybridation in situ fluorescente (FISH) à l'aide de la sonde spécifique pour le gène dans le génome.

5. Utilisation du kit selon l'une quelconque des revendications 1 à 4, dans laquelle lorsque le nombre de copies du gène est supérieur à 4, l'adénocarcinome pulmonaire de stade I chez le mammifère est prédit comme étant un adénocarcinome pulmonaire de stade I de pronostic défavorable.

6. Utilisation du kit selon l'une quelconque des revendications 1 à 5, comprenant en outre la comparaison du niveau d'expression mesuré de la protéine α-actinine-4 dans le tissu ou la cellule avec le niveau d'expression de la protéine α-actinine-4 dans le tissu ou la cellule normal(e) correspondant(e),
**caractérisée en ce que**
lorsque l'expression de la protéine α-actinine-4 est davantage mise en valeur dans le tissu ou la cellule de l'adénocarcinome pulmonaire de stade I par rapport à celle du tissu ou de la cellule normal(e), l'adénocarcinome pulmonaire de stade I chez le mammifère est prédit comme étant un adénocarcinome pulmonaire de stade I de pronostic défavorable.

7. Utilisation du kit selon la revendication 1, dans laquelle le réactif destiné à une détermination quantitative de la protéine α-actinine-4 est un anticorps se liant spécifiquement à la protéine α-actinine-4.

8. Utilisation du kit selon la revendication 1 ou 7, **caractérisée en ce que** la protéine α-actinine-4 présente la séquence d'acides aminés représentée par le SEQ ID N° 2.

9. Utilisation du kit selon l'une quelconque des revendications 1 à 8, comprenant en outre un réactif qui peut mesurer la concentration de l'antigène carcinoembryonnaire (CEA) dans le sérum.

10. Utilisation du kit selon la revendication 9, destinée en outre à comparer la concentration mesurée de l'antigène carcinoembryonnaire (CEA) dans le sérum avec la concentration de l'antigène carcinoembryonnaire (CEA) dans du sérum normal, **caractérisée en ce que**
lorsque l'expression de la protéine α-actinine-4 est davantage mise en valeur dans le tissu ou la cellule de l'adénocarcinome pulmonaire de stade I par rapport à celle du tissu ou de la cellule normal(e), et que la concentration de l'antigène carcinoembryonnaire (CEA) dans le sérum obtenu chez le mammifère est statistiquement et de manière significative supérieure à celle du sérum normal, l'adénocarcinome pulmonaire de stade I chez le mammifère est prédit comme étant un adénocarcinome pulmonaire de stade I de pronostic défavorable.


### SEQUENCE LISTING

<110> Japan Health Sciences Foundation
<120> Method for Prognosis of Early-Stage Pulmonary Adenocarcinoma and Pharmaceutical Composition for Treatment Thereof
<130> 11F012-PCT
<160> 2
<170> PatentIn version 3.3
<210> 1
   <211> 3893
   <212> DNA
   <213> Homo sapiens
<220>
   <221> CDS
   <222> (60)..(2792)
<400> 1
agaggggcgg gagctgaggc gggagcggac aggctggtgg gcgagcgaga ggcggcgga      59
   atg gtg gac tac cac gcg gcg aac cag tcg tac cag tac ggc ccc agc      107
   agc gcg ggc aat ggc gct ggc ggc ggg ggc agc atg ggc gac tac atg      155
   gcc cag gag gac gac tgg gac cgg gac ctg ctg ctg gac ccg gcc tgg      203
   gag aag cag cag cgc aag acc ttc acg gca tgg tgc aac tcc cac ctg      251
   cgg aag gca ggc aca cag atc gag aac att gat gag gac ttc cga gac      299
   ggg ctc aag ctc atg ctg ctc ctg gag gtc ata tca ggg gag cgg tta      347
   cct aag ccg gag cgg ggg aag atg aga gtg cac aaa atc aac aat gtg      395
   aac aaa gcg ctg gac ttt att gcc agc aaa ggc gtc aag ctg gtc tcc      443
   atc ggg gca gaa gag att gtg gac ggc aac gca aag atg acc ctg gga      491
   atg atc tgg acc atc atc ctt agg ttc gcc atc cag gac atc tcc gtg      539
   gaa gag acc tcg gcc aag gaa ggg ctc ctt ctc tgg tgc cag aga aag      587
   aca gcc ccg tat aag aac gtc aat gtg cag aac ttc cac atc agc tgg      635
   aag gat ggt ctt gcc ttc aat gcc ctg atc cac cgg cac aga cca gag      683
   ctg att gag tat gac aag ctg agg aag gac gac cct gtc acc aac ctg      731
   aac aat gcc ttc gaa gtg gct gag aaa tac ctc gac atc ccc aag atg      779
   ctg gat gca gag gac atc gtg aac acg gcc cgg ccc gac gag aag gcc      827
   ata atg acc tat gtg tcc agc ttc tac cat gcc ttt tca gga gcg cag      875
   aag gct gaa act gcc gcc aac cgg atc tgt aag gtg ctg gct gtc aac      923
   caa gag aac gag cac ctg atg gag gac tac gag aag ctg gcc agc gac      971
   ctc ctg gag tgg atc cgg cgc acc atc ccc tgg ctg gag gac cgt gtg     1019
   ccc caa aag act atc cag gag atg cag cag aag ctg gag gac ttc cgc     1067
   gac tac cgg cgt gtg cac aag ccg ccc aag gtg cag gag aag tgc cag     1115
   ctg gag atc aac ttc aac acg ctg cag acc aag ctg cgc ctc agc aac     1163
   cgg ccc gcc ttc atg ccc tcc gag ggc aag atg gtc tcg gac atc aac     1211
   aat ggc tgg cag cac ttg gag cag gct gag aag ggc tac gag gag tgg     1259
   ctg ctg aat gag atc cgc agg ctg gag cgg ctc gac cac ctg gca gag      1307
   aag ttc cgg cag aag gcc tcc atc cac gag gcc tgg act gac ggg aag      1355
   gaa gcc atg ctg aag cac cgg gac tac gag acg gcc aca cta tcg gac      1403
   atc aaa gcc ctc att cgc aag cac gag gcc ttc gag agc gac ctg gct      1451
   gcg cac cag gac cgc gtg gag cag atc gcc gcc att gcc cag gag ctc      1499
   aac gag ctg gat tac tac gac tcc cac aat gtc aac acc cgg tgc cag      1547
   aag atc tgt gac cag tgg gac gcc ctc ggc tct ctg aca cat agt cgc      1595
   agg gaa gcc ctg gag aaa aca gag aag cag ctg gag gcc atc gac cag      1643
   ctg cac ctg gaa tac gcc aag cgc gcg gcc ccc ttc aac aac tgg atg      1691
   gag agc gcc atg gag gac ctc cag gac atg ttc atc gtc cat acc atc      1739
   gag gag att gag ggc ctg atc tca gcc cat gac cag ttc aag tcc acc      1787
   ctg ccg gac gcc gat agg gag cgc gag gcc atc ctg gcc atc cac aag      1835
   gag gcc cag agg atc gct gag agc aac cac atc aag ctg tcg ggc agc      1883
   aac ccc tac acc acc gtc acc ccg caa atc atc aac tcc aag tgg gag      1931
   aag gtg cag cag ctg gtg cca aaa cgg gac cat gcc ctc ctg gag gag      1979
   cag agc aag cag cag tcc aac gag cac ctg cgc cgc cag ttc gcc agc      2027
   cag gcc aat gtt gtg ggg ccc tgg atc cag acc aag atg gag gag atc 2075
   ggg cgc atc tcc att gag atg aac ggg acc ctg gag gac cag ctg agc 2123
   cac ctg aag cag tat gaa cgc agc atc gtg gac tac aag ccc aac ctg 2171
   gac ctg ctg gag cag cag cac cag ctc atc cag gag gcc ctc atc ttc 2219
   gac aac aag cac acc aac tat acc atg gag cac atc cgc gtg ggc tgg 2267
   gag cag ctg ctc acc acc att gcc cgc acc atc aac gag gtg gag aac 2315
   cag atc ctc acc cgc gac gcc aag ggc atc agc cag gag cag atg cag 2363
   gag ttc cgg gcg tcc ttc aac cac ttc gac aag gat cat ggc ggg gcg 2411
   ctg ggg ccc gag gag ttc aag gcc tgc ctc atc agc ctg ggc tac gac 2459
   gtg gag aac gac cgg cag ggt gag gcc gag ttc aac cgc atc atg agc 2507
   ctg gtc gac ccc aac cat agc ggc ctt gtg acc ttc caa gcc ttc atc 2555
   gac ttc atg tcg cgg gag acc acc gac acg gac acg gct gac cag gtc 2603
   atc gct tcc ttc aag gtc tta gca ggg gac aag aac ttc atc aca gct 2651
   gag gag ctg cgg aga gag ctg ccc ccc gac cag gcc gag tac tgc atc 2699
   gcc cgc atg gcg cca tac cag ggc cct gac gcc gtg ccc ggt gcc ctc 2747
   gac tac aag tcc ttc tcc acg gcc ttg tat ggc gag agc gac ctg 2792
   tgaggcccca gagacctgac ccaacacccc cgacggcctc caggaggggc ctgggcagcc 2852
   ccacagtccc attcctccac tctgtatcta tgcaaagcac tctctgcagt cctccggggt 2912
   gggtgggtgg gcagggaggg gctggggcag gctctctcct ctctctcttt gtgggttggc 2972
   caggaggttc ccccgaccag gttggggaga cttggggcca gcgcttctgg tctggtaaat 3032
   atgtatgatg tgttgtgctt ttttaaccaa ggaggggcca gtggattccc acagcacaac 3092
   cggtcccttc catgccctgg gatgcctcac cacacccagg tctcttcctt tgctctgagg 3152
   tcccttcaag gcctccccaa tccaggccaa agccccatgt gccttgtcca ggaactgcct 3212
   gggccatgcg aggggccagc agagggcgcc accaccacct gacggctggg gacccaccca 3272
   gcccctctcc cctctctgct ccagactcac ttgccattgc caggagatgg ccccaacaag   3332
   caccccgctt ttgcagcaga ggagctgagt tggcagaccg ggcccccctg aaccgcaccc   3392
   catcccacca gccccggcct tgctttgtct ggcctcacgt gtctcagatt ttctaagaac 3452
   caaaaaaaaa aaaggaaaaa aaacacaaaa caacaaaaac caaaaaaaaa aaaaatcaca 3512
   aaaacaaaaa aactataaaa aagaaagaat taaaaacttt cagagaatta ctatttactt 3572
   tattaactta cggatttatt atataaatat atattcacct agcaacatat ctctgccgtc 3632
   tctcctgctc tcataatgaa gacatagccg attctctgcc cgggcccctt gctgatgctc 3692
   ctccgggtct gcgtcgggcg tgggtctctg gggaccctcc agaggtggag gtgggctgat 3752
   ggcctggctg cctggtggtt gatggttttg ctccccctac cttttttttt tgagtttatt 3812
   ctgattgatt ttttttcttg gtttctggat aaaccaccct ctggggacag gataataaaa 3872
   catgtaatat ttttaagaag g 3893
<210> 2
   <211> 911
   <212> PRT
   <213> Homo sapiens
<400> 2